# EUROPEAN PATENT SPECIFICATION

(11) **EP 4 643 658 B1**
(45) Date of publication and mention of the grant of the patent: **02.09.2026**
(21) Application number: 24382467.9
(22) Date of filing: 29.04.2024
(51) Int. Cl.: A23L 33/175, A61K 31/195, A61K 31/198, A61K 31/405, A61P 13/12

(54) **DIETARY PRODUCT FOR HYPOPROTEIC DIET**
DIÄTPRODUKT FÜR DIE HYPOPROTEISCHE ERNÄHRUNG
PRODUIT DIÉTÉTIQUE POUR ALIMENTATION HYPOPROTEIQUE

(43) Date of publication of application: 05.11.2025
(73) Proprietor: LABORATORIOS ERN S.A., 08020 Barcelona (ES)
(72) Inventor: SOLANES LÓPEZ, David, BARCELONA (ES)
(74) Representative: Ponti & Partners, S.L.P

(56) References cited:
- WO-A1-2020/244798
- ES-A6- 2 019 248
- FR-A1- 2 615 734
- US-A- 4 100 160
- US-A1- 2018 000 764
- US-A1- 2021 275 479

## Description

### FIELD OF THE INVENTION

The present invention relates to the field of a dietary product for supplementing hypoproteic diet in chronic kidney disease, hepatic insufficiency and urea cycle disorders.

### BACKGROUND ART

Renal insufficiency is characterized by the retention of toxic and nitrogenated metabolites due to the progressive loss of renal function.

Chronic kidney disease (CKD) is a major public health problem worldwide. The goal of CKD treatment is to prevent or slow further damage to the kidneys. Dietary intervention is the main approach in kidney failure. The main solutes retained in CKD are protein-related products that cause hyperazotemia, acidemia, and hyperphosphatemia. Phosphate and sodium also play a relevant role in renal adaptation, causing hyperparathyroidism and extracellular volume expansion, respectively. Generally, nutritional management of CKD requires balancing energy, protein, sodium, potassium, phosphorus, and fluid intake with biochemical markers and weight change.

Markers of kidney damage include: albuminuria greater than or equal to 30 mg/24 h (or ≥30 mg/g depending on albumin/creatinine ratio), abnormalities in urinary sediment, electrolyte concentrations, and other abnormalities due to tubular disorders, histology-detected impairments (i.e., chronic glomerulonephritis) or detected impairments detected by imaging (i.e., polycystic kidneys) or a history of kidney transplantation.

As disclosed in Luft et al., Franz Volhard in Historical Perspective, Hypertension, 1993, 22 (2), 253-256, in the 1930s, Volhard observed the positive effects of a protein-restricted diet on patients with chronic renal failure. The results of these diets was investigated, as disclosed in Giovannetti et al., A low-nitrogen diet with proteins of high biological value for severe chronic uraemia, The Lancet, 1964, 283 (7341), 1000-1003. A diet consisting of a basic caloric regimen with 6-9 g of vegetable protein and 12-15 g of high bio-quality protein extracted from egg protein and a mixture of amino acids was introduced. The use of low-protein diets supplemented with amino acids was an advance in the treatment of uremic symptoms.

This type of diet is based on the control of nitrogen ingested thanks to protein restriction, which leads to a deficit of essential amino acids and stimulates protein catabolism.

A great advance in the therapeutics of uremic symptomatology consists of supplementing protein restriction diets with keto and hydroxy acids (keto and amino acid hydroxy analogues). Keto and hydroxy acids do not contain nitrogen, so they do not produce catabolic nitrogen. Said acids reuse metabolic nitrogen for protein biosynthesis. This process reproduces the process that occurs naturally in the body.

Keto- and hydroxy analogues are precursor compounds of amino acids, which are converted into the amino acids by a transamination reaction and by a combination of oxidation and transamination, respectively. In the transamination reaction, an amino group is removed from an amino acid and transferred to an acceptor ketoacid, and a ketoacid version of the original amino acid is formed. Because keto analogues lack the amino group bound to the alpha carbon of an amino acid, they can be converted to the respective amino acids without additional nitrogen. In the case of hydroxy analogues, an enzymatic oxidation reaction is carried out prior to the subsequent transamination reaction.

The potential benefits of protein restriction with or without supplementation of keto analogues and essential amino acids on the progression of CKD has been the subject of an important debate.

In the review Jiang et al., Effect of restricted protein diet supplemented with keto analogues in chronic kidney disease: a systematic review and meta-analysis, Int. Urol. Nephrol., 2016 48(3), 409-418, it is disclosed that a diet restricted protein supplementation supplemented with keto analogues of amino acids could slow the progression of CKD, decrease hyperphosphatemia, prevent hyperparathyroidism, and improve control of blood pressure without causing malnutrition.

In the review Rhee et al., Low-protein diet for conservative management of chronic kidney disease: a systematic review and meta-analysis of controlled trials, J. Cachexia Sarcopenia Muscle, 2018, 9(2), 235-245, it is disclosed that a low-protein diet seems to improve the treatment of patients with CKD who have not yet need dialysis and may be considered as an option for patients with CKD who wish to avoid or postpone the start of dialysis and slow down the progression of CKD, while the risk of protein energy loss and cachexia remains minimal. In addition, medium-protein diet combined with keto analogue amino acids was associated with greater preservation of renal function and reduction in the rate of progression of end-stage renal disease compared to low-protein diet.

In the review Li et al., The Effect of Ketoanalogues on Chronic Kidney Disease Deterioration: A Meta-Analysis, Nutrients, 2019, 11, 957, it is disclosed that a protein-restricted diet supplemented with keto analogue amino acids significantly delays the progression of CKD.

A dietary supplement for CKD patients, Ernamin^{®}, comprises a combination of amino acids and keto- and hydroxy amino acid analogues, which has a bad taste. This a handicap for patient compliance, which has to take 3 times a day 25 g of the supplement dissolved/dispersed in 200 ml of water.

In ES-A-2019248 it is disclosed a food/dietary product with minimum supply of nitrogen in the form of tablets, which comprises a mixture of diverse essential amino acids, as they are or also substituted by their keto or hydroxy analogues, wherein it has added to it carbohydrates and lipids and corresponds to a general formula made up of 55 to 65% of proteins, 25 to 40% of carbohydrates , 1 to 10% of lipids, and 5 to 10% of food additives. The carbohydrates used are preferably saccharose or fructose and the lipids used consist preferably of soya lecithin. The product is especially suitable for protein compensation in people suffering from renal and / or liver failure, improving the nitrogen balance and favouring anabolism.

In WO-A-2020/244798 it is disclosed that a dietary combination of keto analogue amino acids and some of the scarcest essential amino acids is administered for compensating their deficiency due to low-protein diet or very low-protein diet. It is further disclosed that a problem for the patient is the high amount of supplement to be ingested (in adults, 4 to 8 tablets three times a day in the case of Ketosteril^{®}), and that this administration is cumbersome and uncomfortable, in particular for elderly, persons with cognitive impairment or persons with swallowing problems. Consequently, patient compliance is rather low. This prior art provides a method for taste/odour masking of a liquid oral formulation for CKD patients. Said method is based on the use of three compositions, wherein the first one comprises specific amounts of at least one essential amino acid and/or at least one keto analogue of an essential amino acid and/or at least one hydroxy analogue of an essential amino acid, the second one specific amounts of a combination of hydrogenated and non-hydrogenated phosphatidylcholine and medium-chain triglycerides, and the third one specific amounts of at least one C₂-C₄ alcohol and at least one of glyceryl stearate and/or a saturated or unsaturated C₁₄-C₂₀ fatty acid.

Patients suffering from kidney disease are subjected to fluid restriction, i.e., the intake of liquids are restricted. An excess of fluid intake may result in complications. While healthy kidneys can remove excess fluid from the blood, kidneys with reduced function hold on to excess fluids and waste. Drinking too much fluid can cause swelling and increase your blood pressure, which can lead to complications of the heart. Excess fluid can also build up in the lungs, making it harder to breathe. Symptoms of too much fluid can vary quite significantly. In many cases, symptoms won't be noticed until later, as the body adapts to the situation. Examples of fluid overload symptoms may include: swelling at the feet, ankles, hands, even face; rapid weight gain; headache, cramping; bloating; abdominal pain or discomfort; shortness of breath; hyponatremia (low sodium levels in the blood), or high blood pressure.

The importance of the liquid intake in CKD patients is highlighted in prior art, such as, for example, Clark et al., The Chronic Kidney Disease Water Intake Trial: Protocol of a Randomized Controlled Trial, Can. J. Kidney Health Dis., 2017, 4, 1-11, which discloses a controlled trial to examine the 1-year effect of a higher vs usual water intake on estimated glomerular filtration rate (eGFR) in patients with chronic kidney disease. In Wagner et al., Water intake and progression of chronic kidney disease: the CKD-REIN cohort study, Nephrol. Dial Transplant., 2022, 37, 730-739, the authors concluded that in patients with CKD, the relation between plain water intake and progression to kidney failure appears to be U shaped, i.e., both low and high intake may not be beneficial in CKD.

Thus, in spite of the dietary products disclosed in the prior art, there is a need for further dietary products which provides advantages to the patients subjected to hydric restriction and improves patient compliance.

### SUMMARY OF INVENTION

An aspect of the present invention relates to a dietary product.

Another aspect of the invention is the dietary product for use as supplement of the diet in a method of treatment of patients in need.

### DESCRIPTION OF THE INVENTION

The present invention relates to a dietary product, which comprises:
a) essential amino acids selected from the group consisting of histidine, isoleucine, leucine, lysine, methionine, phenylalanine, threonine, tryptophan, valine, and mixtures thereof,
b) keto and hydroxy analogues of essential amino acids selected from (RS)-3-methyl-2-oxovaleric acid calcium salt, 4-methyl-2-oxovaleric acid calcium salt, 2-oxo-3-phenylpropionic acid calcium salt, 3-methyl-2-oxobutyric acid calcium salt, (RS)-2-hydroxy-4-methylthio-butyric acid calcium salt, and mixtures thereof, and
c) pharmaceutically acceptable excipients,
wherein the pharmaceutically acceptable excipients comprise a combination of medium chain glycerine triesters, Arabic gum, and starch sodium octenyl succinate.

The inventors of the present invention have developed new dietary product providing low nitrogen content for supplementing the diet of patients in need thereof suffering chronic renal failure, liver failure, or congenital disorders of the urea cycle.

The inventors have surprisingly found that the incorporation of medium chain glycerine triesters, Arabic gum, and starch sodium octenyl succinate to the composition improves the organoleptic features of the product, allowing both a higher concentration of the amino acids in the dose and the use of significantly less water for the administration. The consequence of these improvements is a twice per day administration of a product showing better organoleptic features and, thus, ensuring patient compliance.

In the present description, as well as in the claims, the singular forms "a", "an" and "the" include the plural reference unless the context clearly indicates otherwise. The term "about" refers to a deviation of plus/minus 10%, preferably plus/minus 5%. The percentages are expressed in % by weight (wt%), unless stated the contrary. The ranges defined by the terms "between ... and ..." or by the terms "from ...to..." are meant to include also said stated endpoints thereof, and they also include any narrower sub-range.

In the present description, the essential amino acids are L-amino acids, unless stated the contrary.

In the present description, the keto- and hydroxy analogues of essential amino acids bear the keto and the hydroxy groups at the α-position of the carboxylic group.

### Dietary product

The dietary product according to the invention represents a dietary supplement of low-protein diets with minimal nitrogen intake. It lowers the levels of urea and ammonium in the blood, improves uremic symptoms, slows the increase and normalizes creatinine levels in the blood, and promotes a positive nitrogen balance. Additionally, the increased concentration of the amino acids and keto- and hydroxy analogues, the reduced amount of liquid to be used for the administration, and the favourable organoleptic features ensures a higher patient compliance.

The dietary product according to the invention is indicated for chronic renal failure, liver failure, and congenital disorders of the urea cycle.

The dietary product according to the invention is a dietary supplement in which some essential amino acids are replaced by their keto or hydroxy amino acid analogues. These keto-hydroxy amino acid analogues are characterized by having the same hydrocarbon structure as the corresponding amino acid, substituting a nitrogenous group (amino) for an oxygenated one (keto or hydroxy group). With this substitution, the hydrocarbon skeleton necessary for the synthesis of the corresponding essential amino acid from keto-hydroxy analogue is provided to the diet and the retained non-protein nitrogen (urea and ammonium) is reused.

The dietary product according to the invention supplements the low-protein and high-calorie diet with amino acids and amino acid precursors (keto-hydroxy amino acid analogues) with a minimum nitrogen content: 2.6% nitrogen to the total weight of amino acids and amino acid precursors (expressed as bases) compared to 12.1% in an equivalent mixture of essential amino acids. The total nitrogen content is therefore reduced to one-sixth.

The desirability of low-protein diets is based on exogenous nitrogen restriction. The dietary supplement according to the invention with essential amino acids and keto-hydroxy amino acid analogues improves uremic symptomatology, promotes a positive nitrogen balance and favours anabolism.

The dietary product according to the invention provides higher concentration of amino acids and amino acid analogues per dose, significantly reducing the amount of liquid to be daily ingested with the product, and improves patient compliance, even in front of the higher concentration, by appropriate organoleptic features, such as palatability and taste.

The process for preparing the dietary product is a direct mixing of the components, which makes very attractive for industrial manufacturing. The process comprises generally sieving of the components through a 0.5 mm - 1 mm mesh sieve, and mixing in, for example, a V-shaped mixer, which is suitable for free-flowing powders. Mixing time is adjusted according to routine practice to obtain a homogeneous mixture, which is dosed, preferably, in sachets.

Conventional mixing procedures, well-known to the skilled in pharmaceutical technology, are disclosed in reference books in the field, for example, in the book Aulton's Pharmaceutics. The design and manufacture of medicines, M.E. Aulton and K.M.G. Taylor, editors, Churchill Livingstone Elsevier, Fourth Edition, 2013; or in the book Remington: The Science and Practice of Pharmacy, D. Limmer editor, Lippincott Williams & Wilkins, 2000.

### Essential amino acids

The dietary product according to the invention comprises essential amino acids selected from the group consisting of histidine, isoleucine, leucine, lysine, methionine, phenylalanine, threonine, tryptophan, valine, and mixtures thereof.

In a preferred embodiment, the dietary product comprises a mixture of the following essential amino acids: tryptophan, histidine, threonine, and lysine.

The content of tryptophan is usually comprised between 0.3 wt% and 0.6 wt%, preferably between 0.4 wt% and 0.5 wt%, and more preferably about 0.45 wt% over the total weight of the dietary product.

The content of histidine is usually comprised between 0.5 wt% and 0.9 wt%, preferably between 0.6 wt% and 0.8 wt%, and more preferably about 0.75 wt% over the total weight of the dietary product.

The content of threonine is usually comprised between 0.7 wt% and 1.1 wt%, preferably between 0.8 wt% and 1.0 wt%, and more preferably about 0.9 wt% over the total weight of the dietary product.

The content of lysine, expressed as monoacetate, is usually comprised between 0.9 wt% and 1.5 wt%, preferably between 1.0 wt% and 1.4 wt%, and more preferably about 1.25 wt% over the total weight of the dietary product.

### Keto- and hydroxy analogues

The dietary product according to the invention comprises keto and hydroxy analogues of essential amino acids selected from (RS)-3-methyl-2-oxovaleric acid calcium salt (= α-ketoanalogue to DL-isoleucine), 4-methyl-2-oxovaleric acid calcium salt (= α-ketoanalogue to leucine), 2-oxo-3-phenylpropionic acid calcium salt (= α-ketoanalogue to phenylalanine), 3-methyl-2-oxobutyric acid calcium salt (= α-ketoanalogue to valine), (RS)-2-hydroxy-4-methylthio-butyric acid calcium salt (= α-hydroxyanalogue to DL-methionine), and mixtures thereof.

In a preferred embodiment, the dietary product comprises the following keto and hydroxy analogues of essential amino acids (RS)-3-methyl-2-oxovaleric acid calcium salt, 4-methyl-2-oxovaleric acid calcium salt, 2-oxo-3-phenylpropionic acid calcium salt, 3-methyl-2-oxobutyric acid calcium salt, and (RS)-2-hydroxy-4-methylthio-butyric acid calcium salt, which corresponds to a mixture of α-ketoanalogue to DL-isoleucine, α-ketoanalogue to leucine, α-ketoanalogue to phenylalanine, α-ketoanalogue to valine, and α-hydroxyanalogue to DL-methionine, all of them as calcium salts.

The content of 2-oxo-3-phenylpropionic acid (= α-ketoanalogue to phenylalanine), expressed as the calcium salt, is usually comprised between 1.0 wt% and 4.5 wt%, preferably between 2.0 wt% and 3.0 wt%, and more preferably about 2.75 wt% over the total weight of the dietary product.

The content of (RS)-2-hydroxy-4-methylthio-butyric acid (= α-hydroxyanalogue to DL-methionine), expressed as the calcium salt, is usually comprised between 1.0 wt% and 5.0 wt%, preferably between 2.0 wt% and 3.5 wt%, and more preferably about 2.9 wt% over the total weight of the dietary product.

The content of (RS)-3-methyl-2-oxovaleric acid (= α-ketoanalogue to DL-isoleucine), expressed as the calcium salt, is usually comprised between 3.0 wt% and 6.0 wt%, preferably between 3.5 wt% and 5.0 wt%, and more preferably about 4.2 wt% over the total weight of the dietary product.

The content of 4-methyl-2-oxovaleric acid (= α-ketoanalogue to leucine), expressed as the calcium salt, is usually comprised between 4.0 wt% and 6.5 wt%, preferably between 4.5 wt% and 6.0 wt%, and more preferably about 5.3 wt% over the total weight of the dietary product.

The content of 3-methyl-2-oxobutyric acid (= α-ketoanalogue to valine), expressed as the calcium salt, is usually comprised between 4.2 wt% and 6.7 wt%, preferably between 4.7 wt% and 6.3 wt%, and more preferably about 5.5 wt% over the total weight of the dietary product.

### Pharmaceutical excipients

The dietary product according to the invention comprises pharmaceutically acceptable excipients, wherein the pharmaceutically acceptable excipients comprise a combination of medium chain triglycerides (MCT), Arabic gum, and starch sodium octenyl succinate.

Generally, the content of MCT is comprised between 0.05 wt% and 5.0 wt% over the total weight of the dietary product.

Generally, the content of Arabic gum is comprised between 0.01 wt% and 5.0 wt% over the total weight of the dietary product.

Generally, the content of starch sodium octenyl succinate is comprised between 0.05 wt% and 5.0 wt% over the total weight of the dietary product.

The dietary product according to the invention may comprise generally further excipients, such as diluents, flavouring agents, sweetening agents, acidulants, lubricants, binders, glidants, alkalizing agents, buffering agents, antioxidants and mixtures thereof.

Suitable excipients for use in the preparation of the dietary product are described, for example, in the book Handbook of pharmaceutical excipients, R.C. Rowe, P.J. Sheskey and M.E. Quinn, editors, Pharmaceutical Press, sixth edition, 2009.

In an embodiment, the dietary product comprises further excipients selected from the group comprising diluents, flavouring agents, sweetening agents, acidulants, lubricants, binders, glidants, and mixtures thereof.

In a preferred embodiment, the dietary product comprises one or more further excipients selected from diluents, sweetening agents, flavouring agents, and acidulants.

Suitable diluents may comprise, for example, maltodextrin, mannitol, sorbitol, xylitol, isomalt, erythritol, cellulose powdered, microcrystalline cellulose, silicified microcrystalline cellulose, starch, calcium phosphate, calcium lactate, calcium carbonate, magnesium carbonate, magnesium oxide and mixtures thereof; preferably the diluent is maltodextrin.

Generally, the diluent is present in an amount between 10 wt% and 50 wt%, preferably between 20 wt% and 40 wt%, and more preferably between 25 wt% and 30 wt%, over the total weight of the dietary product.

In some embodiments, the dietary product may comprise a sweetening agent, which is usually selected from the group consisting of fructose, sucralose, saccharin sodium, neohesperidin dihydrochalcone, acesulfame potassium, aspartame and mixtures thereof. Preferably the composition does not include sucrose, and therefore it is suitable for diabetics. Generally, the sweetening agent is usually present in an amount from 0.05 wt% to 50 wt% over the total weight of the dietary product composition, depending on the sweetening strength of the sweetening agent. For example, the content of fructose may be between 40 wt% to 50 wt%, whereas the content of sodium saccharin may be between 0.1 wt% and 0.5 wt%, and the content of sucralose may be between 0.05 wt% and 0.15 wt%. In a preferred embodiment, the sweetening agent is a combination of fructose and sodium saccharin. In another preferred embodiment, the sweetening agent further comprises sucralose, it is a combination of fructose, sucralose and sodium saccharin.

The dietary product may further comprise a flavouring agent, wherein it is usually selected from fruit flavours such as banana, strawberry, cherry, wild cherry, lemon, orange, and mixtures thereof, and other flavours such as cardamom, chocolate, anise, peppermint, menthol, cinnamon, vanillin, and mixtures thereof. Generally, the content of the flavouring agent is comprised between 0.05 wt% and 5.0 wt% over the total weight of the dietary product.

In an embodiment, the flavouring agent is selected from the following flavours: banana, strawberry, cherry, wild cherry, lemon, orange, cardamom, chocolate, anise, peppermint, menthol, cinnamon, vanillin, and mixtures thereof.

In a preferred embodiment, the flavouring agent is banana flavour.

In a preferred embodiment, the banana flavour comprises a mixture of isopentyl acetate, 3-methylbutyl butyrate, benzyl isobutyrate, benzyl alcohol, cinnamon essential oil, and cyclohexyl acetate.

In another preferred embodiment, the flavouring agent is vanillin flavour.

In a preferred embodiment, the vanillin flavour comprises a mixture of vanillin, ethyl vanillin, ethyl maltol, cinnamon essential oil, and benzaldehyde.

In an embodiment, the dietary product may further comprise an acidulant, which is usually selected from the group consisting of citric acid, preferably anhydrous, fumaric acid, maleic acid, malic acid, tartaric acid and mixtures thereof; preferably the acidulant is citric acid, more preferably anhydrous. Generally, the content of the acidulant is comprised between 1 wt% and 5 wt% over the total weight of the dietary product.

In an embodiment, the dietary product may further comprise a lubricant, wherein the lubricant is usually selected from the group consisting of talc, sodium benzoate, sodium stearyl fumarate, sodium lauryl sulfate, calcium stearate, magnesium stearate, zinc stearate, glyceryl behenate, stearic acid, glyceryl monostearate, poloxamer, polyethylene glycol and mixtures thereof. Generally, the content of the lubricant is comprised between 0.05 wt% and 5.0 wt% over the total weight of the dietary product.

In an embodiment, the dietary product may further comprise a binder, wherein the binder is usually selected from the group consisting of carbomer, gelatin, guar gum, chitosan, methylcellulose, hypromelose, and mixtures thereof. Generally, the content of the binder is comprised between 0.05 wt% and 5.0 wt% over the total weight of the dietary product.

The dietary product may further comprise a glidant, wherein the glidant is usually selected from the group consisting of colloidal silicon dioxide, calcium phosphate tribasic, hydrophobic colloidal silica, magnesium silicate, magnesium trisilicate, silicon dioxide, talc and mixtures thereof. Generally, the content of the glidant is comprised between 0.05 wt% and 5.0 wt% over the total weight of the dietary product.

The dietary product may further comprise an alkalizing agent, which is usually selected from the group consisting of potassium bicarbonate, potassium citrate, sodium bicarbonate, sodium carbonate and mixtures thereof.

In a preferred embodiment, the dietary product comprises further pharmaceutically acceptable excipients selected from fructose, maltodextrin, sodium saccharin, sucralose, anhydrous citric acid, flavouring agent, and mixtures thereof.

In a preferred embodiment, the pharmaceutically acceptable excipients consist of a mixture of fructose, maltodextrin, sodium saccharin, sucralose, anhydrous citric acid vanillin, ethyl vanillin, ethyl maltol, cinnamon essential oil, and benzaldehyde.

In another preferred embodiment, the pharmaceutically acceptable excipients consist of a mixture of fructose, maltodextrin, sodium saccharin, anhydrous citric acid, isopentyl acetate, 3-methylbutyl butyrate, benzyl isobutyrate, benzyl alcohol, cinnamon essential oil, and cyclohexyl acetate.

### Dietary product

Another aspect of the invention is the dietary product for use as supplement of the diet in a method of treatment of patients suffering from chronic kidney disease, hepatic insufficiency and urea cycle disorders.

In a preferred embodiment, the dietary product is used in combination with a low-protein diet or with a very low-protein diet.

One of the beneficial aspects of treatment with a combination of the dietary product of the invention with a low-protein diet or with a very low-protein diet is that it allows the implementation of dialysis to be delayed, with the advantages that this means for the patient's quality of life and the saving of resources for the health systems. In this regard, both the direct costs of dialysis as well as indirect costs, such as medical transport and other social costs.

The dietary product according to the invention is usually administered twice a day by dissolving/dispersing 25 g of the product in 150 ml of water, providing 6 g/administration of amino acids and amino acids analogues, whereas the prior art product, Ernamin^{®}, is administered 3 times a day by dissolving/dispersing 25 g of the product in 200 ml of water, providing 4 g/administration of amino acids and amino acids analogues.

The dietary product according to the invention allows both a higher concentration of the amino acids in the dose and the use of significantly less water for the daily administration, which is a main issue for patients under hydric restriction. The consequence of these improvements is a twice per day administration of a product showing better organoleptic features and, thus, improving patient compliance by reducing the number of administrations and improving the taste and palatability, and further significantly reducing the daily ingestion of liquids with the product.

### EXAMPLES

### Example 1: Dietary product

A dietary product according to the present invention is shown in Table I:

**TABLE I**

| Component | g/sachet | g/day |
|---|---|---|
| L-Tryptophan | 0.113 | 0.226 |
| L-Histidine | 0.184 | 0.368 |
| L-Threonine | 0.229 | 0.458 |
| L-Lysine monoacetate | 0.308 | 0.616 |
| α-ketoanalogue to phenylalanine, calcium salt | 0.690 | 1.380 |
| α-hydroxyanalogue to DL-methionine, calcium salt | 0.724 | 1.448 |
| α-ketoanalogue to DL-isoleucine, calcium salt | 1.058 | 2.116 |
| α-ketoanalogue to leucine, calcium salt | 1.324 | 2.648 |
| α-ketoanalogue to valine, calcium salt | 1.373 | 2.746 |
| Fructose | 11.347 | 22.694 |
| Maltodextrin | 7.050 | 14.100 |
| Citric acid anhydrous | 0.500 | 1.000 |
| Sodium saccharin | 0.050 | 0.100 |
| Flavour | 0.010 | 0.020 |
| Arabic gum | 0.010 | 0.020 |
| MCT | 0.010 | 0.020 |
| Modified starch | 0.020 | 0.040 |
| Total (g) | 25.000 | 50.000 |
| Total amino acids (g) | 6.000 | 12.00 |

The flavour consisted of a combination of isopentyl acetate, 3-methylbutyl butyrate, benzyl isobutyrate, benzyl alcohol, cinnamon essential oil, and cyclohexyl acetate.

The dietary product was prepared by thoroughly mixing all the solid components to obtain a homogenized mixture and adding the liquid components thereto.

This dietary product may be administered twice a day dissolving/dispersing the 25 g of a sachet in 150 ml of water, resulting a total of 300 ml of water per day, which is a favourable situation for a patient under hydric restriction.

### Example 2: Dietary product

A dietary product according to the present invention is shown in Table II:

**TABLE II**

| Component | g/sachet | g/day |
|---|---|---|
| L-Tryptophan | 0.113 | 0.226 |
| L-Histidine | 0.184 | 0.368 |
| L-Threonine | 0.229 | 0.458 |
| L-Lysine monoacetate | 0.308 | 0.616 |
| α-ketoanalogue to phenylalanine, calcium salt | 0.690 | 1.380 |
| α-hydroxyanalogue to DL-methionine, calcium salt | 0.724 | 1.448 |
| α-ketoanalogue to DL-isoleucine, calcium salt | 1.058 | 2.116 |
| α-ketoanalogue to leucine, calcium salt | 1.324 | 2.648 |
| α-ketoanalogue to valine, calcium salt | 1.373 | 2.746 |
| Fructose | 11.277 | 22.554 |
| Maltodextrin | 7.070 | 14.140 |
| Citric acid anhydrous | 0.500 | 1.000 |
| Sodium saccharin | 0.050 | 0.100 |
| Sucralose | 0.020 | 0.040 |
| Flavour | 0.010 | 0.020 |
| Arabic gum | 0.010 | 0.020 |
| MCT | 0.040 | 0.080 |
| Modified starch | 0.020 | 0.040 |
| Total (g) | 25.000 | 50.00 |
| Total amino acids (g) | 6.000 | 12.00 |

This dietary product according to the present invention was prepared analogously to that of Example 1, with the difference that the flavour was a combination of vanillin, ethyl vanillin, ethyl maltol, cinnamon essential oil, and benzaldehyde, and the presence of sucralose.

This dietary product may be administered twice a day dissolving/dispersing the 25 g of a sachet in 150 ml of water, resulting a total of 300 ml of water per day, which is a favourable situation for a patient under hydric restriction.

### Comparative Example 1: Dietary product of prior art

A dietary product of prior art (Ernamín^{®} Sabor Naranja) is shown in Table III:

**TABLE III**

| Component | g/sachet | g/day |
|---|---|---|
| L-Tryptophan | 0.075 | 0.225 |
| L-Histidine | 0.123 | 0.368 |
| L-Threonine | 0.153 | 0.458 |
| L-Lysine monoacetate | 0.205 | 0.615 |
| α-ketoanalogue to phenylalanine, calcium salt | 0.460 | 1.380 |
| α-hydroxyanalogue to DL-methionine, calcium salt | 0.483 | 1.448 |
| α-ketoanalogue to DL-isoleucine, calcium salt | 0.705 | 2.115 |
| α-ketoanalogue to leucine, calcium salt | 0.883 | 2.648 |
| α-ketoanalogue to valine, calcium salt | 0.915 | 2.745 |
| Fructose | 11.000 | 33.000 |
| Maltodextrin | 7.000 | 21.000 |
| Citric acid anhydrous | 0.500 | 1.500 |
| Sodium saccharin | 0.050 | 0.150 |
| Mannitol | 1.248 | 3.744 |
| Flavour | 1.000 | 3.000 |
| Soja lecithin | 0.200 | 0.600 |
| Dyestuff | 0.002 | 0.006 |
| Total (g) | 25.000 | 75.000 |
| Total amino acids (g) | 4.000 | 12.000 |

This dietary product from prior art was administered three times a day dissolving/dispersing the 25 g of a sachet in 200 ml of water, resulting a total volume of 600 ml of water per day, which is not a favourable situation for a patient under hydric restriction.

### Example 3: Organoleptic comparison

The organoleptic features of the dietary product of Examples 1 and 2, according to the present invention, were tested comparative to a prior art dietary product of Comparative Example 1.

Organoleptic tests of the compositions were carried out in 7 healthy volunteers.

The methodology for evaluating the acceptance of the compositions by the patient consisted in establishing a score from 1 to 5 for palatability of the formulation, where 1 corresponds to an unacceptable palatability, and 5 corresponds to an excellent palatability, and another score from 1 to 5 to assess the taste of the formulation, wherein 1 corresponds to an unacceptable taste, and 5 corresponds to an excellent taste. The multiplication of the palatability score of the composition by the value referring to the taste, gives rise to a value that is the degree of acceptance of the composition by the patient.

The dietary products of Examples 1 and 2, according to the invention, had a score of 4 in both parameters, giving an acceptance of 16.

The Prior art dietary product of Comparative Example 1 had a score of 3 in both parameters, giving an acceptance of 9. The volunteers were unanimous selecting the dietary product according to the invention.

In a test with 33 patients (men 67%, mean age 68 ± 10 years) for 9 months, 80% of the patients showed a mean or high adherence to the dietary product according to the invention.

## Claims

1. A dietary product, which comprises:
a) essential amino acids selected from the group consisting of histidine, isoleucine, leucine, lysine, methionine, phenylalanine, threonine, tryptophan, valine, and mixtures thereof,
b) keto and hydroxy analogues of essential amino acids selected from (RS)-3-methyl-2-oxovaleric acid calcium salt, 4-methyl-2-oxovaleric acid calcium salt, 2-oxo-3-phenylpropionic acid calcium salt, 3-methyl-2-oxobutyric acid calcium salt, (RS)-2-hydroxy-4-methylthio-butyric acid calcium salt, and mixtures thereof, and
c) pharmaceutically acceptable excipients,
wherein the pharmaceutically acceptable excipients comprise a combination of medium chain triglycerides, Arabic gum, and starch sodium octenyl succinate.

2. The dietary product according to claim 1, wherein it comprises a mixture of the following essential amino acids: tryptophan, histidine, threonine, and lysine.

3. The dietary product according to claim 1 or 2, wherein it comprises the following keto and hydroxy analogues of essential amino acids (RS)-3-methyl-2-oxovaleric acid calcium salt, 4-methyl-2-oxovaleric acid calcium salt, 2-oxo-3-phenylpropionic acid calcium salt, 3-methyl-2-oxobutyric acid calcium salt, and (RS)-2-hydroxy-4-methylthio-butyric acid calcium salt.

4. The dietary product according to any one of claims 1 to 3, wherein it comprises one or more further excipients selected from diluents, sweetening agents, flavouring agents, acidulants, lubricants, and glidants.

5. The dietary product according to claim 4, wherein the diluent is selected from maltodextrin, mannitol, sorbitol, xylitol, isomalt, erythritol, cellulose powdered, microcrystalline cellulose, silicified microcrystalline cellulose, starch, calcium phosphate, calcium lactate, calcium carbonate, magnesium carbonate, magnesium oxide and mixtures thereof; preferably the diluent is maltodextrin.

6. The dietary product according to claim 4, wherein the sweetening agent is selected from the group consisting of fructose, sucralose, saccharin sodium, neohesperidin dihydrochalcone, acesulfame potassium, aspartame and mixtures thereof.

7. The dietary product according to claim 6, wherein the sweetening agent is a combination of fructose and sodium saccharin.

8. The dietary product according to claim 7, wherein the sweetening agent further comprises sucralose.

9. The dietary product according to claim 4, wherein the flavouring agent is selected from the following flavours: banana, strawberry, cherry, wild cherry, lemon, orange, cardamom, chocolate, anise, peppermint, menthol, cinnamon, vanillin, and mixtures thereof.

10. The dietary product according to claim 4, wherein the acidulant is selected from the group consisting of citric acid, preferably anhydrous, fumaric acid, maleic acid, malic acid, tartaric acid and mixtures thereof; preferably the acidulant is citric acid, more preferably anhydrous.

11. The dietary product according to claim 4, wherein it comprises further pharmaceutically acceptable excipients selected from maltodextrin as diluent, fructose, sodium saccharin and sucralose as sweetening agents, anhydrous citric acid as acidulant, flavouring agent, and mixtures thereof.

12. The dietary product according to claim 11, wherein the further pharmaceutically acceptable excipients consist of a mixture of maltodextrin, fructose, sodium saccharin, sucralose, anhydrous citric acid, and banana flavour.

13. The dietary product according to claim 11, wherein the further pharmaceutically acceptable excipients consist of a mixture of maltodextrin, fructose, sodium saccharin, anhydrous citric acid, and vanillin flavour.

14. Dietary product according to any one of claims 1 to 13, for use as supplement of the diet in a method of treatment of patients suffering from chronic kidney disease, hepatic insufficiency and urea cycle disorders.

15. Dietary product for use according to claim 14, wherein the use of the dietary product is in combination with a low-protein diet or with a very low-protein diet.

## Patentansprüche

1. Ernährungsprodukt, das umfasst:
a) essentielle Aminosäuren, ausgewählt aus der Gruppe bestehend aus Histidin, Isoleucin, Leucin, Lysin, Methionin, Phenylalanin, Threonin, Tryptophan, Valin und Mischungen davon,
b) Keto- und Hydroxy-Analoga von essenziellen Aminosäuren, ausgewählt aus (RS)-3-Methyl-2-oxovaleriansäure-Calciumsalz, 4-Methyl-2-oxovaleriansäure-Calciumsalz, 2-Oxo-3-phenylpropionsäure-Calciumsalz, 3-Methyl-2-oxobuttersäure-Calciumsalz, (RS)-2-Hydroxy-4-methylthiobuttersäure-Calciumsalz und Mischungen davon, und
c) pharmazeutisch verträgliche Hilfsstoffe,
wobei die pharmazeutisch verträglichen Hilfsstoffe eine Kombination von mittelkettigen Triglyceriden, Gummi arabicum und Stärkenatriumoctenylsuccinat umfassen.

2. Ernährungsprodukt nach Anspruch 1, wobei es eine Mischung der folgenden essentiellen Aminosäuren umfasst: Tryptophan, Histidin, Threonin und Lysin.

3. Ernährungsprodukt nach Anspruch 1 oder 2, wobei es die folgenden Keto- und Hydroxy-Analoga von essenziellen Aminosäuren umfasst: (RS)-3-Methyl-2-oxovaleriansäure-Calciumsalz, 4-Methyl-2-oxovaleriansäure-Calciumsalz, 2-Oxo-3-phenylpropionsäure-Calciumsalz, 3-Methyl-2-oxobuttersäure-Calciumsalz und (RS)-2-Hydroxy-4-methylthio-buttersäure-Calciumsalz.

4. Ernährungsprodukt nach einem der Ansprüche 1 bis 3, wobei es einen oder mehrere weitere Hilfsstoffe umfasst ausgewählt aus Verdünnungsmitteln, Süßungsmitteln, Aromastoffen, Säuerungsmitteln, Gleitmitteln und Fließregulierungsmitteln .

5. Ernährungsprodukt nach Anspruch 4, wobei das Verdünnungsmittel ausgewählt ist aus Maltodextrin, Mannitol, Sorbitol, Xylitol, Isomalt, Erythritol, Cellulosepulver, mikrokristalliner Cellulose, silifizierter mikrokristalliner Cellulose, Stärke, Calciumphosphat, Calciumlactat, Calciumcarbonat, Magnesiumcarbonat, Magnesiumoxid und Mischungen davon; vorzugsweise ist das Verdünnungsmittel Maltodextrin.

6. Ernährungsprodukt nach Anspruch 4, wobei das Süßungsmittel aus der Gruppe ausgewählt ist, die aus Fruktose, Sucralose, Natriumsaccharin, Neohesperidin-Dihydrochalkon, Acesulfam-Kalium, Aspartam und Mischungen davon besteht.

7. Ernährungsprodukt nach Anspruch 6, wobei das Süßungsmittel eine Kombination von Fruktose und Natriumsaccharin ist.

8. Ernährungsprodukt nach Anspruch 7, wobei das Süßungsmittel ferner Sucralose umfasst.

9. Ernährungs produkt nach Anspruch 4, wobei der Aromastoff aus den folgenden Aromen ausgewählt ist: Banane, Erdbeere, Kirsche, Sauerkirsche, Zitrone, Orange, Kardamom, Schokolade, Anis, Pfefferminze, Menthol, Zimt, Vanillin und Mischungen davon.

10. Ernährungsprodukt nach Anspruch 4, wobei das Säuerungsmittel aus der Gruppe ausgewählt ist, die aus Zitronensäure, vorzugsweise wasserfrei, Fumarsäure, Maleinsäure, Apfelsäure, Weinsäure und Mischungen davon besteht; vorzugsweise ist das Säuerungsmittel Zitronensäure, weiter bevorzugt wasserfrei.

11. Ernährungsprodukt nach Anspruch 4, wobei es ferner pharmazeutisch verträgliche Hilfsstoffe umfasst, ausgewählt aus Maltodextrin als Verdünnungsmittel, Fruktose, Natriumsaccharin und Sucralose als Süßungsmittel, wasserfreier Zitronensäure als Säuerungsmittel, Aromastoffen und Mischungen davon.

12. Ernährungsprodukt nach Anspruch 11, wobei die weiteren pharmazeutisch verträglichen Hilfsstoffe aus einer Mischung aus Maltodextrin, Fruktose, Natriumsaccharin, Sucralose, wasserfreier Zitronensäure und Bananenaroma bestehen.

13. Ernährungsprodukt nach Anspruch 11, wobei die weiteren pharmazeutisch verträglichen Hilfsstoffe aus einer Mischung aus Maltodextrin, Fruktose, Natriumsaccharin, wasserfreier Zitronensäure und Vanillineroma bestehen.

14. Ernährungsprodukt nach einem der Ansprüche 1 bis 13 zur Verwendung als Ernährung bei einem Verfahren zur Behandlung von Patienten, die an chronischer Nierenerkrankung, Leberinsuffizienz und Harnstoffzyklusstörungen leiden.

15. Ernährungsprodukt zur Verwendung nach Anspruch 14, wobei das Ernährungsprodukt in Kombination mit einer proteinarmen Ernährung oder einer sehr proteinarmen Ernährung verwendet wird.

## Revendications

1. Un produit diététique, qui comprend :
a) des acides aminés essentiels choisis dans le groupe constitué par l'histidine, l'isoleucine, la leucine, la lysine, la méthionine, la phénylalanine, la thréonine, le tryptophane, la valine, et des mélanges de ceux-ci,
b) des analogues céto et hydroxy d'acides aminés essentiels choisis parmi le sel de calcium de l'acide (RS)-3-méthyl-2-oxovalérique, le sel de calcium de l'acide 4-méthyl-2-oxovalérique, le sel de calcium de l'acide 2-oxo-3-phénylpropionique, le sel de calcium de l'acide 3-méthyl-2-oxobutyrique, le sel de calcium de l'acide (RS)-2-hydroxy-4-méthylthio-butyrique, et des mélanges de ceux-ci, et
c) des excipients pharmaceutiquement acceptables,
dans lequel les excipients pharmaceutiquement acceptables comprennent une combinaison de triglycérides à chaîne moyenne, de gomme arabique, et d'octénylsuccinate sodique d'amidon.

2. Le produit diététique selon la revendication 1, dans lequel il comprend un mélange des acides aminés essentiels suivants : tryptophane, histidine, thréonine, et lysine.

3. Le produit diététique selon la revendication 1 ou 2, dans lequel il comprend les analogues céto et hydroxy d'acides aminés essentiels suivants : sel de calcium de l'acide (RS)-3-méthyl-2-oxovalérique, sel de calcium de l'acide 4-méthyl-2-oxovalérique, sel de calcium de l'acide 2-oxo-3-phénylpropionique, sel de calcium de l'acide 3-méthyl-2-oxobutyrique, et sel de calcium de l'acide (RS)-2-hydroxy-4-méthylthio-butyrique.

4. Le produit diététique selon l'une quelconque des revendications 1 à 3, dans lequel il comprend un ou plusieurs excipients supplémentaires choisis parmi des diluants, des agents édulcorants, des agents aromatisants, des acidulants, des lubrifiants, et des agents d'écoulement.

5. Le produit diététique selon la revendication 4, dans lequel le diluant est choisi parmi la maltodextrine, le mannitol, le sorbitol, le xylitol, l'isomalt, l'érythritol, la cellulose en poudre, la cellulose microcristalline, la cellulose microcristalline silicifiée, l'amidon, le phosphate de calcium, le lactate de calcium, le carbonate de calcium, le carbonate de magnésium, l'oxyde de magnésium et des mélanges de ceux-ci ; de préférence le diluant est la maltodextrine.

6. Le produit diététique selon la revendication 4, dans lequel l'agent édulcorant est choisi dans le groupe constitué par le fructose, le sucralose, la saccharine sodique, la néohespéridine dihydrochalcone, l'acésulfame potassique, l'aspartame et des mélanges de ceux-ci.

7. Le produit diététique selon la revendication 6, dans lequel l'agent édulcorant est une combinaison de fructose et de saccharine sodique.

8. Le produit diététique selon la revendication 7, dans lequel l'agent édulcorant comprend en outre du sucralose.

9. Le produit diététique selon la revendication 4, dans lequel l'agent aromatisant est choisi parmi les arômes suivants : banane, fraise, cerise, cerise sauvage, citron, orange, cardamome, chocolat, anis, menthe poivrée, menthol, cannelle, vanilline, et des mélanges de ceux-ci.

10. Le produit diététique selon la revendication 4, dans lequel l'acidulant est choisi dans le groupe constitué par l'acide citrique, de préférence anhydre, l'acide fumarique, l'acide maléique, l'acide malique, l'acide tartrique et des mélanges de ceux-ci ; de préférence l'acidulant est l'acide citrique, plus préférablement anhydre.

11. Le produit diététique selon la revendication 4, dans lequel il comprend des excipients pharmaceutiquement acceptables supplémentaires choisis parmi la maltodextrine en tant que diluant, le fructose, la saccharine sodique et le sucralose en tant qu'agents édulcorants, l'acide citrique anhydre en tant qu'acidulant, un agent aromatisant, et des mélanges de ceux-ci.

12. Le produit diététique selon la revendication 11, dans lequel les excipients pharmaceutiquement acceptables supplémentaires consistent en un mélange de maltodextrine, de fructose, de saccharine sodique, de sucralose, d'acide citrique anhydre, et d'arôme de banane.

13. Le produit diététique selon la revendication 11, dans lequel les excipients pharmaceutiquement acceptables supplémentaires consistent en un mélange de maltodextrine, de fructose, de saccharine sodique, d'acide citrique anhydre, et d'arôme de vanilline.

14. Produit diététique selon l'une quelconque des revendications 1 à 13, pour utilisation comme supplément du régime alimentaire dans un procédé de traitement de patients souffrant de maladie rénale chronique, d'insuffisance hépatique et de troubles du cycle de l'urée.

15. Produit diététique pour utilisation selon la revendication 14, dans lequel l'utilisation du produit diététique est en combinaison avec un régime alimentaire pauvre en protéines ou avec un régime alimentaire très pauvre en protéines.
